# EUROPEAN PATENT SPECIFICATION

(11) **EP 1 635 661 B1**
(45) Date of publication and mention of the grant of the patent: **11.08.2010**
(21) Application number: 04736720.6
(22) Date of filing: 11.06.2004
(51) Int. Cl.: A41D 13/11, B01D 39/14, A62B 7/10, B01D 71/00, B01D 39/16

(54) **PRODUCT FOR ABSORPTION PURPOSES**
PRODUKT FÜR ABSORPTIONSZWECKE
PRODUIT DESTINE A L'ABSORPTION

(30) Priority: 13.06.2003 US 477960 P
(43) Date of publication of application: 22.03.2006
(73) Proprietor: WADSTRÖM, Torkel, S-224 67 Lund (SE); Ljung, Asa, 224 67 Lund (SE); Hjertén, Stellan, S-752 52 Uppsala (SE)
(72) Inventor: WADSTRÖM, Torkel, 224 67 Lund (SE); LJUNG, Asa, 224 67 Lund (SE); HJERTEN, Stellan, 756 52 Uppsala (SE); ILBÄCK, Nils-Gunnar, 752 27 Uppsala (SE); HJERTEN, Marie-Christine, 754 42 Uppsala (SE)
(74) Representative: Johansson Webjörn, Ingmari
(86) International application number: PCT/SE2004/000928
(87) International publication number: WO 2004/110193

(56) References cited:
- EP-A1- 1 064 979
- EP-A1- 1 470 083
- EP-A2- 0 433 661
- EP-1- 1 444 996
- WO-A1-94/00166
- WO-A1-03/033053
- WO-A1-03/064330
- WO-A2-01/08792
- US-A- 4 411 795
- US-A1- 2001 034 055
- US-A1- 2003 168 401
- DATABASE WPI Week200452, Derwent Publications Ltd., London, GB; Class A96, AN 2004-538059, XP002981458 & JP 2004 204401 A (EBARA CORP) 22 July 2004

## Description

The present invention relates to a product for absorption purposes, preferably for the absorption of airborne and/or liquid borne microbes as well as viruses, and microbial antigens including allergens (which may be fungal), comprising an in water insoluble support matrix which is connected to a hydrophobic entity which in turn is connected to a positively charged entity (other than said in water insoluble support matrix). As a support matrix an organic polymer or a combination of such e. g. polysaccharides such as cellulose etc, may be used. The present invention aims for achieving an improved absorbent which binds, preferably airborne and/or liquid borne, microorganisms such as bacteria as well as viruses, which also preferably may be airborne and/or liquid borne, and/or allergens.

### Background

An increasing problem of airborne microbes and viruses (e.g. Influenza and SARS) and microbial antigens in airborne infections and associated diseases such as asthma has encouraged the development of a more effective method to remove these agents and antigens (i) from a highly contaminated environment, (ii) environments with conventional air filters, such as hospital operating theatres and hospital ward rooms for severely immuno-suppressed patients, and (iii) by personal face masks for hospital personnel. Such new equipment would be of great importance in modem hospital care with numerous patients highly sensitive for infections, e.g. in hematology, oncology and transplantation units.

The present invention discloses, among other things, products, e.g. barriers/filters, to trap airborne and/or liquid borne bacteria, viruses and fungi, for which no efficient such barriers/filters exist, for protecting patients, hospital personnel and people in general during epidemics. It can also be applied on surgical equipment and in showers for immuno-suppressed patients.

EP-A2-0 433 661 relates to polymeric microfiber filter medium comprising a fibrous matrix to whose surfaces is grafted a superstrate comprising a polymer with quaternary ammonium groups and made from an ethylenically unsaturated monomer containing a quaternary ammonium or amino group, the filter medium having a positive zeta potential at pH 7 and preferably an enhanced Latex Adsorption Capacity together with low extractables and quick rinse-up.

WO 01/08792 relates to charged filtration membranes and their use for separation of a protein from solvent, low molecular weight solutes or a mixture of proteins. Modification of the membranes to generate charge includes modification of membrane pores to alter charge within a pore and alter the size of a pore. Consequently, the protein is separated from other solutes in a mixture based on size as well as net protein charge and membrane charge.

There have been filters disclosed in US 4,883,052, US 5,817,584, US 6,412,486, US 6,119,691 and US 4,985,280, but no one of the disclosed filters makes use of the fact that all microbes are negatively charged and that most pathogenic microbes and viruses express strong or moderate cell surface hydrophobicity and accordingly these filters provide less efficient absorption of said microbes and viruses. The very same principle may be used to trap airborne and/or liquid borne allergens. Accordingly, it would be useful with new more efficient absorbing materials using the combination of facts as set out above i.e. that all microbes and viruses are negatively charged and that most pathogenic microbes and viruses express strong or moderate cell surface hydrophobicity.

### Summary of the invention

These objects, are achieved and further advantages are obtained with a product according to the invention as claimed in claim 1, a method of its manufacture according to claim 4, and its use according to claim 5. According to a preferred aspect of the present invention there is also provided a face mask. According to a preferred of the present invention there is also provided a wound dressing (compress) According to a preferred aspect of the present invention there is also provided a drape for use during a surgical intervention According to a preferred aspect of the present Invention there is also provided a filter. The filter is then both hydrophobic and positively charged. According to a preferred aspect of the present invention there is also provided a "tea bag", preferably for obtaining potable water by dipping said tea bag into non-potable water . According to a preferred aspect of the present invention there is also provided a nasal spray for capturing microorganisms, preferably airborne and/or liquid borne microorganisms, as well as viruses, preferably airborne and/or liquid borne viruses in the nasal cavity. According to a preferred aspect of the present invention there is also provided an ointment for capturing microorganisms, preferably airborne and/or liquid borne microorganisms, as well as viruses, preferably airborne and/or liquid borne viruses on the skin of animals or humans. Said ointment may in addition to said product according to claim 1 comprise other components for use In ointments and said additional components for use in ointments are known for a person skilled in the art. According to a preferred aspect of the present invention there is also provided medical devices, e.g. catheters for drainage and rinsing of the urinary and genital tracts. Preferably said device is a catheter which may be used in the urinary, genital or respiratory tracts. According to a preferred aspect of the present invention there is also provided filter arrangement comprising two filters previously mentioned wherein said filters are having in between them one or more products according to claim 1 in particulate form, thus enabling a larger surface area for absorption. According to a preferred aspect of the present invention there is also provided a food wrapping and an active food packaging material comprising a product according to claim 1 said product may preferably comprise in its support matrix a polysaccharide in its native state, positively charged.

### Detailed description of the invention

It is intended throughout the present description that the expression "support matrix" embraces any matrix which is built up of an in water insoluble polymer material. Examples thereof are agarose particles, agar particles and polygalactanes (comprising polygalactose units), agarose or derivatives thereof, laminarine, cellulose (e. g. cotton) or derivatives thereof, cross-linked dextrane or derivatives thereof, and starch or derivatives thereof. Preferably materials commonly used in filter or face masks are used as support matrix. An example thereof is cellulose, which is the most preferred. A polysaccharide such as agarose and cellulose may be regarded as thread-shaped molecules consisting of monomer units containing several hydroxyl groups and internal and external ether bonds (acetal bonds), which taken together give the polysaccharide affinity to water (it is said to be hydrophilic). Such polymers form In water swellable gels with hydroxyls as targets for substitution. It is also possible to use a polymeric backbone (preferably comprising polyethylene) covered with cellulose or similar material as a support matrix and this is especially preferred when using the absorption product according to the first aspect of the invention in face mask filters (the use of a backbone makes it easier to breath in said face mask) or in wound dressings. The support matrix may further be present in particulate form allowing the application of the product for absorption purposes according to the first or third aspect of the present invention by means of a nasal spray or an ointment.

According to a further embodiment of the first aspect of the present invention there is provided a product wherein the hydrophobic entity is a saturated or unsaturated hydrocarbon chain with a chain length of from C₅ to C₂₅ or an aromatic group i.e. an alkyl or alkylene with a chain length of from C₅ to C₂₅, preferably with a chain length of from C₈ to C₁₈, most preferred a saturated or unsaturated hydrocarbon chain with a chain length of from C₁₂ to C₁₈. The chain length may e.g. be C₁₅. The hydrocarbon chain may be included in a compound such as QUAB 342 (see below).

According to the present invention, there is provided a product wherein the positively charged entity is a positively charged group. The ammonium group is contained in a compound such as QUAB 342 (3-chloro-2-hydroxypropyl-dimethyl-dodecylammonium chloride)

According to a further embodiment of the present invention there is provided a product wherein the support matrix comprises a polysaccharide, polygalactane, agar, agarose, laminarine, cellulose, crosslinked dextran, starch or a derivative thereof; or a mixture of two or more of said compounds; preferably said support matrix comprises cellulose.

According to a further embodiment of the present invention there is provided a product wherein the support matrix comprises a backbone covered with cellulose, preferably said backbone comprises a plastic material, most preferred polyethylene. The backbone may consist essentially of polyethylene only. Said product is especially useful in face mask filters (the use of a backbone makes it easier to breath in said face mask) or in wound dressings.

According to the invention the product is used for absorbing microorganisms, preferably airborne and/or liquid borne microorganisms, as well as viruses, preferably airborne (in particular influenza viruses, SARS-virus) and/or liquid borne viruses, and also allergens, the airborne and/or liquid borne microorganisms may be wound pathogens such as *Staphylococcus aureus,* Group A beta-haemolytic streptococci, urinary catheter-related pathogens such as Escherichia coli, eczema-related pathogens such as *Candida albicans* and various bacteria , and burn pathogens such as Pseudomonas aeruginosa. Moreover, "new" pathogens prevalent particularly in the hospital setting are of interest, including methicillin-resistant *Staphylococcus aureus* (MRSA), vancomycin-resistant enterococci (VRE), *Acinetobacter* spp., multiresistant gramnegative intestinal bacteria, *Stenotrophomonas maltophilia* etc.

These microorganisms express high surface hydrophobicity and have been shown to bind to hydrophobic wound dressings.

It should be noted and recalled that all microbes as well as viruses are negatively charged and most pathogenic microbes as well as viruses express strong or moderate cell surface hydrophobicity. The same principle may be used to trap airborne and/or liquid borne allergens. A further advantage with the present invention is the possibility of capturing liquid borne microorganisms such as bacteria as well as viruses, which also preferably may be liquid borne, efficiently even if there is a high concentration of salts in the liquid where the microorganisms such as bacteria as well as viruses reside. Thus said liquid may have a varying content of salts ranging from virtually zero (water) to liquids comprising high concentration of salts. Said liquid may further be a buffer.

Depending on a great variety of applications for this new air-capture (for water applications, see below) different filters can be produced with varying filtering and absorbing capacities per gram material. The product of the first aspect may further be optimized for its binding capacity, e.g. when used in face masks humified by the normal breath. Similar products of the first aspect of the invention may further be used for water to capture water borne microbes, as well as viruses and microbial and/or viral lysate products. Similar products of the first aspect of the invention may further be used food wrapping and active food packaging material for binding and absorbing contaminating bacteria in food, including microbial toxins and bacterial lysate products. Products according to the first or third aspects may also be used for absorbing bacteria and/or viruses and/or fungi in open wounds. These wound may be present on animals and on humans.

Preferred features of each aspect of the invention are as for each of the other aspects mutatis mutandis. The prior art documents mentioned herein are incorporated to the fullest extent permitted by law. The invention is further described in the following examples, which also refers to figures, which do not limit the scope of the invention in any way. Embodiments of the present invention are thus described in more detail with the aid of examples of embodiments (together with figures), the only purpose of which is to illustrate the invention and are in no way intended to limit its extent.

### Figures

Fig.1 shows density of *Staphylococcus aureus* in washing solution (H₂O) after use for washing a QUAB 342-non-treated control filter previously dipped in a solution containing 5x10⁷/ml of *Staphylococcus aureus.*
Fig. 2 shows density of *Staphylococcus aureus* In washing solution (H₂O) after use for washing a QUAB 342-treated filter previously dipped in a solution containing 5x10⁷/ml of *Staphylococcus aureus.*
Fig. 3 shows density of *Escherichia coli* in washing solution (H₂O) after use for washing a QUAB 342-non-treated control filter previously dipped in a solution containing 5x10⁷/ml of *Escherichia coli.*
Fig. 4 shows density of *Escherichia coli* in washing solution (H₂O) after use for washing a QUAB 342-treated filter previously dipped in a solution containing 5x10⁷/ml of *Escherichia coli.*

### Examples

### Example 1: Bacterial adsorption

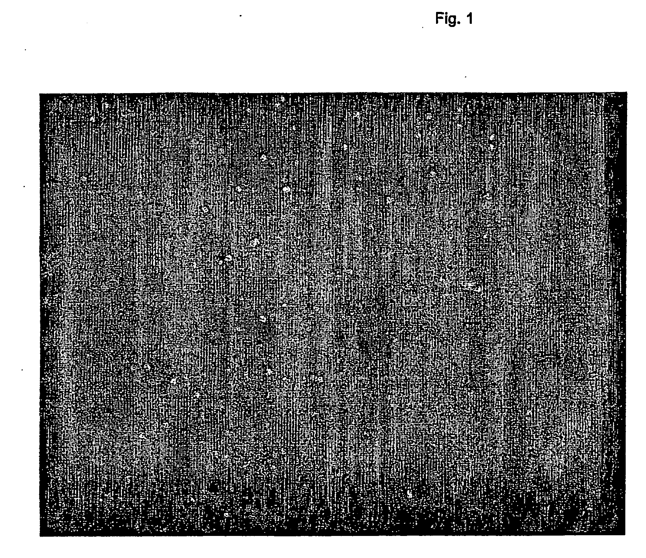

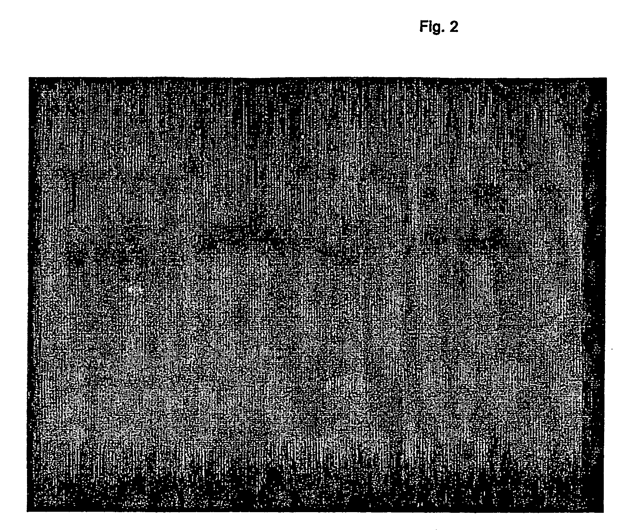

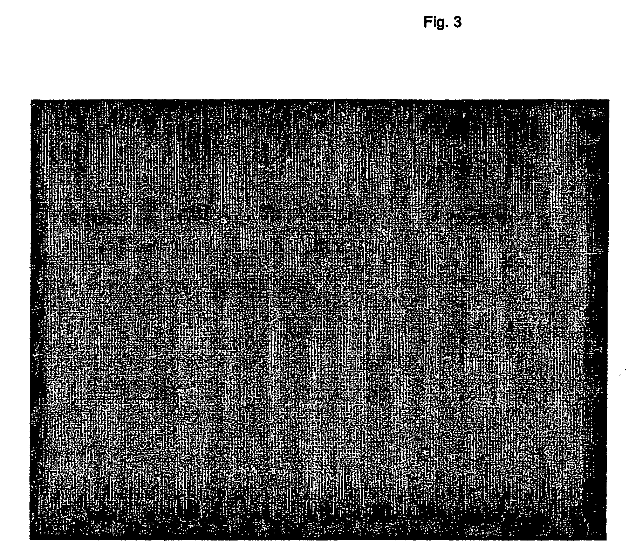

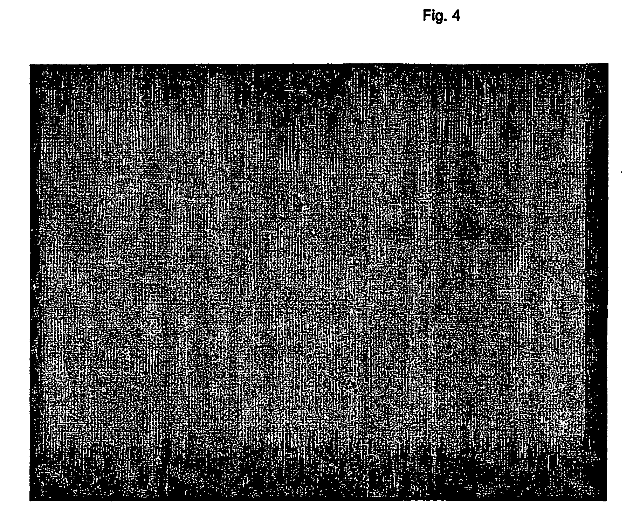

Pieces of non-treated and treated (QUAB 342) cellulose filters with a surface area of about 0.5 cm² were dipped during 1 min in solutions of different bacteria (5x10⁷/ml), i.e., *Escherichia coli* and *Staphylococcus aureus.* After dipping, the filters were washed during 1 min in 2 ml de-ionized water. Bacteria in the washing solution were stained with acridine-orange and quantitatively evaluated using fluorescence microscopy. Results showed that both bacteria were efficiently bound by the treated filters when compared to non-treated filters (see Figures 1 - 4).

### Example 2: Adsorption of virus to cellulose filters

Pieces of non-treated and treated (QUAB 342) cellulose filters were dipped and washed as in experiments A and B. After dipping and washing, the non-treated filters retained 36% (H₂O wash), whereas QUAB 342-treated filters retained 80% (H₂O wash) of the labelled virus. The experiment in A was thus repeated with the difference that the filters were not treated with DACC but QUAB 342 (a compound which has both a hydrophobic and a positively charged group and a reactive Cl atom) for 30 hours and they were then washed for 5 minutes 4 times with 50 ml of water and dried over night. The filters were then as above dipped into a [³⁵S] -methionine/cysteine labeled Corona virus solution. The radioactivity was 2202 cpm.

In addition, a blocking experiment was performed to show the specificity of the binding of virus. Non-treated and treated filters were pre-dipped in non-labelled virus solution prior to dipping in the labelled virus solution. After dipping in labelled virus solution, the filters were washed as previously. After this blocking experiment, the non-treated filter retained 24%, whereas the QUAB 342-treated filter retained 57% of the labelled virus. In conclusion, a clear-cut blocking effect by the non-labelled virus was shown, verifying the specificity of the binding.

Further similar experiments, involving virus, have been performed which gave similar results as the ones given above.

Various embodiments of the present invention have been described above but a person skilled in the art realizes further minor alterations, which would fall into the scope of the present invention. The breadth and scope of the present invention should not be limited by any of the above-described exemplary embodiments, but should be defined only in accordance with the following claims and their equivalents. For example, any of the above-noted products and/or methods can be combined with known therapies for treating microorganisms and/or viruses or compositions/products. Also any of the above-noted products and/or methods can be utilized in other areas than that of microorganisms and allergens for the removal of undesired particles and molecules. Other aspects, advantages and modifications within the scope of the invention will be apparent to those skilled in the art to which the invention pertains.

## Claims

1. A product for absorption purposes consisting of an in water insoluble support matrix wherein the support matrix is connected to a hydrophobic entity which in turn is connected to a positively charged entity, other than said in water insoluble support matrix, wherein 3-chloro-2-hydroxypropyl-dimethyl-dodecyl-ammonium chloride is attached to the support matrix.

2. A product according to claim 1, wherein the support matrix comprises a polysaccharide, polygalactane, agar, agarose, laminarine, cellulose, crosslinked dextran, starch or a derivative thereof; or a mixture of two or more of said compounds; preferably said support matrix comprises cellulose.

3. A product according to any of the preceding claims, wherein the support matrix comprises a backbone covered with cellulose, preferably said backbone comprises a plastic material, most preferred said backbone comprises polyethylene.

4. A method for the manufacture of a product according to any one of the preceding claims, wherein the hydrophobic entity connected to a positively charged entity, is attached to the support matrix wherein 3-chloro-2-hydroxypropyl-dimethyl-dodecyl-ammonium chloride is attached to the support matrix.

5. Use of a product according to any one of claims 1 to 3 for absorbing microorganisms, preferably airborne and/or liquid borne microorganisms, as well as viruses, preferably airborne and/or liquid borne viruses, and/or allergens.

6. A face mask comprising a product according to any one of claims 1 to 3.

7. A wound dressing comprising a product according to any one of claims 1 to 3.

8. A drape for use during a surgical intervention, comprising a product according to any one of claims 1 to 3.

9. A filter comprising a product according to any one of claims 1 to 3.

10. A tea bag comprising a product according to any one of claims 1 to 3.

11. A nasal spray comprising a product according to any one of claims 1 to 3.

12. An ointment comprising a product according to any one of claims 1 to 3.

13. A medical device comprising a product according to claim 1 to 3, preferably said device is a catheter.

14. A filter arrangement comprising two filters according to claim 9 wherein said filters are having in between them one or more products according to claim 1 to 3 in particulate form.

15. A food wrapping or food packaging material comprising a product according to claim 1 to 3.

## Patentansprüche

1. Produkt für Absorptionszwecke, bestehend aus einer in Wasser unlöslichen Trägermatrix, wobei die Trägermatrix mit einer hydrophoben Einheit verbunden ist, die ihrerseits mit einer positiv geladenen Einheit, die von der in Wasser unlöslichen Trägermatrix verschieden ist, verbunden ist, wobei an der Trägermatrix 3-Chlor-2-hydroxypropyldimethyldodecyl-ammoniumchlorid befestigt ist.

2. Produkt gemäß Anspruch 1, wobei die Trägermatrix ein Polysaccharid, Polygalactan, Agar, Agarose, Laminarin, Cellulose, vernetztes Dextran, Stärke oder ein Derivat davon oder ein Gemisch von zwei oder mehr dieser Verbindungen umfasst, wobei die Trägermatrix vorzugsweise Cellulose umfasst.

3. Produkt gemäß einem der vorstehenden Ansprüche, wobei die Trägermatrix ein mit Cellulose bedecktes Rückgrat umfasst, wobei das Rückgrat vorzugsweise ein Kunststoffmaterial umfasst, wobei das Rückgrat höchst bevorzugt Polyethylen umfasst.

4. Verfahren zum Herstellen eines Produkts gemäß einem der vorstehenden Ansprüche, wobei die hydrophobe Einheit, die mit einer positiv geladenen Einheit verbunden ist, an der Trägermatrix befestigt ist, wobei an der Trägermatrix 3-Chlor-2-hydroxypropyldimethyl-dodecylammoniumchlorid befestigt ist.

5. Verwendung eines Produkts gemäß einem der Ansprüche 1 bis 3 zum Absorbieren von Mikroorganismen, vorzugsweise luftübetragenen und/oder flüssigkeitsübertragenen Mikroorganismen, sowie von Viren, vorzugsweise luftübetragenen und/oder flüssigkeitsübertragenen Viren, und/oder Allergenen.

6. Gesichtsmaske, umfassend ein Produkt gemäß einem der Ansprüche 1 bis 3.

7. Wundverband, umfassend ein Produkt gemäß einem der Ansprüche 1 bis 3.

8. Vorhang zur Verwendung während eines chirurgischen Eingriffs, umfassend ein Produkt gemäß einem der Ansprüche 1 bis 3.

9. Filter, umfassend ein Produkt gemäß einem der Ansprüche 1 bis 3.

10. Teebeutel, umfassend ein Produkt gemäß einem der Ansprüche 1 bis 3.

11. Nasenspray, umfassend ein Produkt gemäß einem der Ansprüche 1 bis 3.

12. Salbe, umfassend ein Produkt gemäß einem der Ansprüche 1 bis 3.

13. Medizinische Einheit, umfassend ein Produkt gemäß Anspruch 1 bis 3, wobei die Einheit vorzugsweise ein Katheter ist.

14. Filteranordnung, umfassend zwei Filter gemäß Anspruch 9, wobei die Filter zwischen sich ein oder mehr Produkte gemäß Anspruch 1 bis 3 in Teilchenform aufweisen.

15. Nahrungsmitteleinwickel- oder Nahrungsmittelverpackungsmaterial, umfassend ein Produkt gemäß Anspruch 1 bis 3.

## Revendications

1. Produit destiné à des fins d'absorption constitué d'une matrice support insoluble dans l'eau dans lequel la matrice support est reliée à une entité hydrophobe qui à son tour est reliée à une entité chargée positivement, autre que ladite matrice support insoluble dans l'eau, du chlorure de 3-chloro-2-hydroxypropyldiméthyldodécylammonium étant fixé à la matrice support.

2. Produit selon la revendication 1, dans lequel la matrice support comprend un polysaccharide, un polygalactane, de l'agar-agar, de l'agarose, de la laminarine, de la cellulose, du dextrane réticulé, de l'amidon ou un dérivé de celui-ci ; ou un mélange de deux ou plus de deux desdits composés ; de préférence ladite matrice support comprend de la cellulose.

3. Produit selon l'une quelconque des revendications précédentes, dans lequel la matrice support comprend une structure de base recouverte de cellulose, de préférence ladite structure de base comprend une matière plastique, de façon préférée par-dessus tout ladite structure de base comprend du polyéthylène.

4. Procédé pour la fabrication d'un produit selon l'une quelconque des revendications précédentes, dans lequel l'entité hydrophobe reliée à une entité chargée positivement est fixée à la matrice support, du chlorure de 3-chloro-2-hydroxypropyldiméthyldodécyl-ammonium étant fixé à la matrice support.

5. Utilisation d'un produit selon l'une quelconque des revendications 1 à 3 pour l'absorption de microorganismes, de préférence de microorganismes présents dans l'air et/ou présents dans un liquide, ainsi que de virus, de préférence de virus présents dans l'air et/ou présents dans un liquide, et/ou d'allergènes.

6. Masque facial comprenant un produit selon l'une quelconque des revendications 1 à 3.

7. Pansement comprenant un produit selon l'une quelconque des revendications 1 à 3.

8. Champ opératoire destiné à être utilisé pendant une intervention chirurgicale, comprenant un produit selon l'une quelconque des revendications 1 à 3.

9. Filtre comprenant un produit selon l'une quelconque des revendications 1 à 3.

10. Sachet de thé comprenant un produit selon l'une quelconque des revendications 1 à 3.

11. Pulvérisation nasale comprenant un produit selon l'une quelconque des revendications 1 à 3.

12. Pommade comprenant un produit selon l'une quelconque des revendications 1 à 3.

13. Dispositif médical comprenant un produit selon la revendication 1 à 3, de préférence ledit dispositif étant un cathéter.

14. Agencement de filtres comprenant deux filtres selon la revendication 9 dans lequel lesdits filtres ont entre eux un ou plusieurs produits selon la revendication 1 à 3 sous forme particulaire.

15. Matériau d'enveloppement alimentaire ou d'emballage alimentaire comprenant un produit selon la revendication 1 à 3.
